# EUROPEAN PATENT APPLICATION

(11) **EP 3 731 236 A1**
(43) Date of publication of application: **28.10.2020**
(21) Application number: 19170613.4
(22) Date of filing: 23.04.2019
(51) Int. Cl.: G16H 30/20, G16H 50/20, G16H 50/30

(54) **METHOD OF EVALUATING MR IMAGES FOR SEDATION MONITORING OF A PATIENT BY MR IMAGING**

(71) Applicant: Koninklijke Philips N.V., 5656 AG Eindhoven (NL)
(72) Inventor: WEISS, Steffen, 5656 AE Eindhoven (NL); HELLE, Michael Günter, 5656 AE Eindhoven (NL)
(74) Representative: de Haan, Poul Erik

(57) **Abstract**

A method 100 of evaluating MR images for sedation monitoring by MR imaging is presented which is capable of reducing manual labor by acquiring information about the sedation level of a patient out of MR images.

## Description

### FIELD OF THE INVENTION

The invention is directed to a method for evaluating MR images for sedation monitoring by MR imaging, a computer program element, a computer readable medium and an MR imaging device for sedation monitoring.

### BACKGROUND OF THE INVENTION

The process of sedation monitoring during Magnetic Resonance (MR) imaging is quite a complex and labor-intensive process with current available systems. Typically, sedation monitoring is done by staff in form of patient response to verbal or tactile stimulus. There are also several types of tactile and verbal response systems to monitor the sedation of the patient during MR imaging. Tactile and verbal responses can be evaluated by medical staff, which are a cost factor in MR imaging. Furthermore, the medical staff mainly has to enter the MR room, what can have negative influence on the MR imaging. Additionally, every patient can form an individual response, which must be interpreted by the medical staff. This can decrease the objectiveness of the evaluation of the sedation of the patient.

### SUMMARY OF THE INVENTION

The object of the present invention may be seen in an improvement of a sedation monitoring during MR imaging.

The problem is solved by a method for evaluating MR images for sedation monitoring by MR imaging according to claim 1 and the subject matter of the other independent claims. Further embodiments and advantages of the present invention are incorporated in the dependent claims and the description.

Technical terms are used herein by common sense. If a specific meaning is conveyed to certain terms, definitions of terms will be given in the following in the context of which the terms are used.

According to a first aspect of the present invention, a method of evaluating MR images for sedation monitoring of a patient by MR imaging comprises the steps of:
- providing an MR image of a body part,
- determining a perfusion of at least one part of the body part in the MR image,
- calculating a change of the perfusion of the at least one part of the body part,
- generating a signal indicative of an alteration of a sedation of the patient based on the calculated change of the perfusion of the at least one part of the body part.

An advantage of such a method is that with the help of the determination of the perfusion in a body part, the sedation level of the patient within the MR imaging can be monitored without attaching sensors or awaiting tactile or verbal feedback. Furthermore, labor can be reduced, since the medical staff does not need to enter the MR room to receive the patient verbal and/or tactile responses. Furthermore, the presented method may increase the global numbers of MR examinations since there is a lack of experienced staff in many regions.

Anaesthetic-induced thermoregulatory inhibition is dose-dependent and impairs the vasoconstriction of the body part. By determining the perfusion within MR images the sedation level of the patient can be assessed. Therefore, with the help of this method it is possible to monitor the sedation level of the patient irrespective of the type of patient or sedation medicament used.

Furthermore, with the help of the determination of the perfusion in the body part, the onset of the sedation of the patient can be detected much earlier after an anesthetic agent was administered in comparison to conventional indicators like the loss of feeling cold, pain and/or any haptic feedback. For example, the onset of sedation of the patient can be assessed already after 12 minutes since an anesthetic agent has been administered by determining the change of perfusion in the body part. In comparison, the patient cannot feel pain in said body part after e.g. 24 minutes after the anesthetic agent has been administered. Accordingly, the time period to be able to detect a sedation of the patient can be reduced by determining the perfusion in MR images. Furthermore, the method is variable to different examination conditions, for example, the method can be applied at different scans or even during execution of certain sequences that present longer acquisition times.

In other words, the method for sedation monitoring presented herein can comprise the step of providing an MR image of a body part. A body part can be any part of a human body or any part of any animal or organism. The MR image can be provided e.g. from a storage device, like for example RAM, hard disk, virtual RAM, virtual hard disk or any other storage device capable of storing MR images, a database, a cloud database, a PACS system and/or can be acquired live. The MR image can be e.g. a T1-, T2- and/or PD-weighted MR image or any other MR-image in which a perfusion of the examined body part can be determined. Furthermore, the method comprises the step of determining a perfusion of at least one part of the body part in the MR image. In particular, the perfusion can be a blood perfusion within a vessel or any other body part through which blood flows. Additionally, the body part can be any part of a body and any part of the body part. For example in case the perfusion is assessed in a hand of the patient, the perfusion can also be determined in parallel in a finger of the hand. Furthermore, the body part can be an arm, a hand, a finger, a leg, a foot, a toe and/or a nose.

Furthermore, the method comprises the step of calculating a change of the perfusion of at least one part of the body part. A change of the perfusion can be any curve, diagram, or any other indicator. Furthermore, the change can be embodied as a single measurement point, a plurality of measurement points, in particular a chain of measurement points. For example, the perfusion can comprise at least two measurement points, which can form a curve, which can be e.g. perfusion over time. A first derivative can be formed from the curve to determine the change of the perfusion. Additionally, the change of the perfusion can be expressed in a three dimensional diagram, which can be a combination of perfusion and dosage over time. For example, the change may be an analysis of the perfusion, in particular the vasoconstriction of vessels, which is monitored.

Furthermore, the method comprises the step of generating a signal indicative of an alteration of a sedation of the patient based on the change of perfusion from its normal level, and/or from a calculated change of the perfusion of the at least one part of the body part. In other words, the sedation of the patient is determined with the help of this change and/ or change of the perfusion. In particular, an increase or decrease of the perfusion can be correlated to the sedation level of the patient. For example, a raise of the perfusion in the body part can be correlated to an increase of the sedation of the patient. Also, in case the perfusion decreases in the body part, the perfusion decrease can be correlated to a decrease of the sedation level of the patient.

Furthermore, a signal can be generated to alert supervisory personnel or to alter a sedation of the patient with the help of a narcotic agent dispenser, which is able to inject a narcotic agent automatically. Alternatively, the signal can also be inputted into any other form of device, which can generate an alert or indicate an alteration of the sedation of the patient to supervisory personnel.

According to exemplary embodiment of the present invention, the perfusion is determined in thermoregulatory vessels, in particular in arterio-venos shunts, of the body part.

In other words, with the help of the method, the perfusion within thermoregulatory vessels can be determined with the help of MR imaging. Thereby, any other form of monitoring can be avoided so that less labour and equipment is necessary in order to be able to monitor a sedation level of the patient during MR imaging. The body dissipates most of its metabolic heat via the skin surface. The main mechanism to limit this heat loss is by cutaneous vasoconstriction, resulting in a minimization of flow of warm blood through the skin. Therefore, vasoconstriction is the most consistently used autonomous mechanism to regulate body temperature. This establishes a temperature gradient from body core to periphery under normal conditions. Only in the event of excess heat production, typically by physical work, vasoconstriction is relaxed to allow more dissipation of heat from the skin. To accomplish this mechanism the skin comprises two types of blood vessels: nutritional vessels and thermoregulatory vessels. By monitoring thermoregulatory vessels, the perfusion of the body part can be assessed reliably.

Additionally the perfusion in arterio-venos shunts of the body part can be measured, which is determined by assessing vasoconstriction in the arterio-venos shunts. The method can provide the determination of the perfusion in an arteriovenous shunt by calculating the change of the perfusion within the arteriovenous shunt to be able to monitor a sedation level, which is based on the vasoconstriction in the arteriovenous shunt. The advantage of this embodiment can be that the sedation level can be assessed much more quickly than with the help of conventional sedation level monitoring.

According to an exemplary embodiment of the present invention, the perfusion is determined in a peripheral area of the body part, through which blood flows, in particular a tissue of the body part. In other words, the perfusion can be determined in a tissue of the body part in which thermoregulatory vessels are located. The peripheral area of the body part can be for example the dermis (skin) of a body part. The advantage of this embodiment can be that the perfusion can be assessed more reliable since the peripheral area of the body part can be the area in which the perfusion changes promptly and the change can be easily assessed.

According to an exemplary embodiment of the present invention, the determination of the perfusion in the body part can be repeated and recorded over time, and wherein the step of determining the perfusion further comprises:
- generating a baseline, which is a reference for the determination of the perfusion,
- defining at least one limitation of the change based on the baseline,
- comparing the change to the at least one limitation of the change,
- generating the signal indicative of the alteration of the sedation of the patient if the calculated change exceeds the at least one limitation of the change.

In other words, a method is provided to monitor the change of the perfusion within the body part to be able to generate the signal alerting the supervisory personnel if the change exceeds a limitation of the perfusion. To be able to apply this method on a broad variety of body parts, a baseline can be set based on the body part and the person being examined with the help of the presented method. The baseline can also be a reference point within the time development of the perfusion. Additionally, the perfusion is recorded over time, for example in a diagram comprising two axis wherein one axis is the perfusion and the other is the time. Furthermore, a limitation of the change of the perfusion can be defined based on the baseline or in dependency of the baseline. For example, the limitation can be a value at which the level of the sedation of the patient becomes critical. Comparing the change to the at least one limitation of the change can happen constantly or during certain time points, for example, every 0,5 - 10 minutes. The time-evolution of the perfusion can serve as surrogate for the time-evolution of the sedation depth. In many cases, the patient may have been brought to the correct level of sedation at the beginning of the MR examination. Therefore, the baseline may be generated at the beginning of the MR examination.

The MR examination may start with the acquisition of baseline that will typically last less than 1 min. Additionally the acquisition of the baseline can be repeated in order to generate a more reliable starting point in order to determine the change of the perfusion. The acquisition of the baseline can trigger further acquisitions of the perfusion scan, in particular in predetermined time intervals, for example between 0,5 and 10 minutes. Most MR examinations can comprise diagnostic scans that each last between 2 and 10 min. Further sub-minute perfusion scans may repeatedly be interleaved with those scans. Longer diagnostic scans may be interrupted to interleave a perfusion scan. The rate at which perfusion scans may be interleaved with diagnostic scans can be chosen to match the typical time scales at which the sedative state of the patient changes, i.e. in the order of 4-10min. This may be required to adequately sample the time-evolution of sedative depth.

Furthermore, the method is suggested to generate a signal, which can be an electronic or acoustic and/or a visual signal that is indicative for an alteration of the sedation level of the patient when the calculated change exceeds the limitation of the change. Exceeding of the limitation of the change can be just for a short time, wherein the limitation can comprise a buffer such that no signal is generated. If the change exceeds the limitation for a longer time period, the signal can be generated. The advantage of that embodiment can be that with the help of the comparison of the change to the limitation of the change, the sedation level can be controlled independently of a knowledge level of the operator and thereby increasing the reliability of the sedation monitoring.

According to an exemplary embodiment of the present invention, the determination of the perfusion in the body part can be recorded over time, wherein the step of determining the perfusion may further comprise:
- generating a curve, which describes the perfusion of the body part over time;
- evaluating a first derivative of the generated curve in order to determine a change of the perfusion in the body part.

In other words, the perfusion can be recorded over time such that a curve is generated describing the perfusion of the body part over time, for example, wherein the first axis can be the perfusion and a second axis can be the time. By evaluating the first derivative of the generated curve, a change of the perfusion can be assessed. The change of the perfusion can be indicative for a change of a sedation level of a patient. This embodiment can comprise the advantage that with this embodiment processing power can be saved, since the sedation level can be assed with energy saving calculations.

According to an exemplary embodiment of the present invention, the method can further comprise the steps of:
- comparing the change with a predetermined threshold value, and
- generating the signal indicative of the alteration of the sedation of the patient if the calculated change exceeds the determined threshold value.

In other words, a predetermined threshold value is defined which can stand for a maximum or minimum perfusion which can be expressed through the change. The predetermined threshold value can also stand for a maximum or minimum change over time of the change and/or the perfusion within the body part. If the maximum or minimum change of the predetermined threshold value is exceeded by the calculated change, a signal is generated indicative of the alteration of the sedation of the patient. This embodiment can comprise the benefit that with a simple method, the sedation level of a body part can be analyzed so that personnel usage of medically trained staff can be reduced and cost can be saved.

According to an exemplary embodiment of the present invention, the method further comprises:
- performing blood oxygen level dependent imaging (BOLD), wherein a result of BOLD is interpreted in order to determine the perfusion.

In other words, the results of BOLD can be interpreted such that the perfusion, in particular changes of the perfusion, in the body part can be determined. The blood oxygen level dependent imaging can be in particular performed on thermoregulatory vessels, arteriovenous shunts and/or in superficial tissue of the body part. A variant of the typical BOLD imaging sequence as used for brain functional magnetic resonance imaging (fMRI) may be proposed. The conventional sequence can use an EPI read-out with a very high number of echoes and an echo time similar to the T2* value of blood (e.g. about 30-40 ms @ 3T). Often one axial slice across the entire brain may be acquired in a single shot, typically in less than Is. Several slices can be acquired in interleaved fashion, depicting a stack of slices across the brain with a temporal resolution tailored to the typical time scales in which neurovascular blood supply changes upon brain activation being in the order of 5s-15s. In standard BOLD, the patient performs certain tasks in a repetitive on/off pattern, and the temporal correlation of the time-varying BOLD signal in sync with this pattern is used to identify activated regions.

In order to determine the perfusion in the body part, the BOLD imaging slice or a small imaging volume can have a small field-of-view, typically involving about 20 instead of more than 64-128 phase encodings. Moreover, perfusion can be not expected to change within 5-10s time-scales but be about constant over 1 min. Therefore, a higher number of signal averages per slice (order of 100 for a lmin single slice scan) may be acquired to increase SNR, or small volumes should be imaged which intrinsically also increases SNR. The blood flow typically may increase by about 30% upon brain activation resulting in a signal change in the order of 1 to 2% in conventional brain fMRI. In the proposed method, skin perfusion may increase considerably more resulting in a larger signal change.

According to an exemplary embodiment of the present invention, the method comprises the step:
- performing arterial spin labelling (ASL) for the step of determining the perfusion.

In other words, arterial spin labelling is performed on the human body part and/or the MR image in order to determine the perfusion in the body part. This embodiment can have the advantage that the signal to noise ratio (SNR) can be increased and therefore the quality of determining the perfusion can be increased. The blood oxygen level dependent imaging and the arterial spin labelling can be performed in sequence, in parallel or alternatively only one of both can be performed in order to determine the perfusion.

ASL imaging sequences can be grouped into pulsed (PASL) and continuous (CASL) labelling techniques, the latter also with the variant PCASL, meaning pseudo-continuous labelling. In PASL a very short (10-20 ms) labelling pulse can be applied to a 10-20 cm thick slab proximally (towards the heart) next to the imaging zone, and imaging may be started after some of this blood has entered the imaging zone. In (P)CASL a long (1-2s) long labelling may be applied to a thin slice next to the imaging zone so that further blood can be labelled while some labelled blood already enters this zone. For full brain imaging the amount of labelled blood entering the imaging zone can be larger in (P)CASL than in PASL resulting in higher SNR. Additionally, for (P)CASL the average delay between labelling of some blood and imaging of this blood may be shorter than in PASL. Because labelling may fade out with T1 decay this can translate into a larger ASL signal in (P)CASL.

For the perfusion application, it is possible to use PCASL together with the three-dimensional MR images. The smaller imaging field-of-view may be smaller in phase-encoding direction and also by fewer slices than in full brain imaging results in a shorter imaging period, and it is proposed to use this time for additional signal averaging. In combination with two-dimensional MR images, the CASL approach maybe used because together with correct choice of labelling slab thickness and delay between labelling and imaging, substantial averaging can be applied which increases SNR.

Another well suited perfusion measurement technique named velocity-selective ASL (VSASL) can be employed for the determining of the perfusion. VSASL may not require a spatial selectivity to label the spins in the arterial blood outside of the imaging volume. Instead, blood can be labelled according to the flow velocity. Therefore, VSASL can provide quantitative measures especially under slow flow conditions present in peripheral body parts.

Artefacts due to motion between the labelling and the control experiment can be a major source of error in ASL. Techniques for background suppression (BS) have been developed to mitigate this and may be used here for two- and/or three-dimensional MR-images. BS may prepare the magnetization of the imaging region such that it's signal may be already minimal in a unsubtracted images. Because the imaging times can be shorter here than in full brain ASL due to small field-of-view, motion artefacts can be expected to be generally reduced. In addition, it is proposed to perform motion-corrected averaging: Individual images both for two-dimensional and three-dimensional MR images may be registered with each other before averaging is performed.

According to an exemplary embodiment of the present invention, the provided MR image is a two-dimensional MR image of the body part, and wherein the two-dimensional MR image is orientated perpendicular to a main orientation of the body part.

In other words, the MR imaging plane is orientated perpendicular to the main orientation of the body part, for example a finger. In particular, when the slice is orientated perpendicular, e.g. to a longitudinal axis of a finger, the finger is displayed in a form of circles in the two-dimensional image. This orientation of the slice can reduce the scan time drastically and the effort in image segmentation can be reduced.

Preferentially, the slice may be located through and perpendicular to the direction of several fingers, several toes, or the tip of the nose. Phase-encoding direction can be chosen into the direction where the slice does not intersect any other parts of the body. Especially for the hands lying right and left beside the body, this will may be the anterior-posterior direction. This can be essential to achieve a low number of phase-encodings and thus lower scan time. The slice thickness can be chosen in the order of 4 mm, so that there may be little anatomic structure across the imaging slice. The in-plane resolution can be chosen in the order of 2 mm to depict the superficial perfusion effect well. Due to the above explained choice of the MR image slice location and orientation, from which the perfusion is determined, the image content may be relatively well-defined and therefore computing power can be saved since the image segmentation requires less effort. It may consist of several circles side by side, representing the cross-sections of the slice with the fingers or toes, and one circle in case of the nose. Despite major motion between several perfusion scans should be rare due to sedation, it may be still proposed to perform a segmentation of the region of interest within the image. This can be important to always select corresponding regions for determining the perfusion, what may result in a consistent time curve. In the most simple case segmentation can be performed by identification land marks of the body part by thresholding. Subsequently, a ring-like region with a width reaching until the depth maybe selected where perfusion changes are known to take place.

According to an exemplary embodiment of the present invention, the provided MR image is a three-dimensional MR image of the body part, wherein the three-dimensional MR image is orientated perpendicular to a feeding vessel, in particular a head to foot direction of a hand and/or a foot or in anterior or posterior direction of a nose.

This embodiment can comprise the advantage that a perfusion within the three-dimensional MR image can be determined more reliably. Especially for perfusion imaging in the hand or foot, a true 3D-encoded imaging volume consisting of several slices containing all fingers and part of the palm or the entire fore-foot may be used. For the hands, read-out direction can be chosen primarily in left-right direction to be able to clip signal originating from the trunk. Especially in the ASL imaging case, the slice direction can be perpendicular to the flow direction of the blood from which the perfusion can be determined, i.e. in feet-head direction for hands, oblique for feet, and in anterior-posterior direction for the nose. For the image processing of three-dimensional MR images at first the outer hull of the hand or foot can be assessed by thresholding. In a next step, a shell reaching into the hand/foot with a thickness matching that of typical perfusion changes may be segmented. Furthermore, a labeling plane of the ASL should be essentially in parallel to a imaging plane of the ASL. Additionally the labeling plane should positioned at a distance from the imaging plane of the ASL, in particular at a distance against the direction of the blood flow from which the perfusion is determined. In other words, the labeling plane of the ASL should be located closer to the heart of the patient than the imaging plane of the ASL.

According to an exemplary embodiment of the present invention, the method comprises segmenting the body part. The segmentation can be performed in order to determine the perfusion and/or in order to detect a motion of the body part. In particular, the segmentation can serve to have a reliable perfusion measurement throughout the complete MR imaging process. Additionally, the segmentation can serve to base the perfusion measurement on those regions where actual changes happen. This can result in a more reliable determining of the perfusion since a consistent curve of the perfusion over time can be generated.

According to an exemplary embodiment of the present invention, a sequence of MR images is provided. Additionally, the method can comprise the step of automatically detecting a motion of the body part by analyzing the sequence of the MR images. In other words, the method is capable of detecting a motion of a body part, in particular a finger or the like within a MR image and/or a sequence of MR images. The detection of the motion can be taken into account to determine a change in the perfusion and thereby a change in a sedation level of the patient.

According to an exemplary embodiment of the present invention, the sequence of MR images comprises at least a first MR image and a second MR image. Furthermore, segmenting the body part in the first MR image and in the second MR image thereby determining a position of the body part within the first MR image and the second MR image. Additionally the method can comprise a comparing of the position of the body part in first MR image to the position of body part in the second MR image. The method can comprise the step of generating a signal indicative for a motion of the body part.

In other words, a motion of the body part can be detected. The detection of the motion of the body part can have the advantage that an artefact in the determination of the perfusion can be interrelated to a motion of the body part. The motion can be detected performing a segmentation of the body part in each of the MR images in the sequence. After the segmentation, the positon of the body part can be identified in each of the MR images. The relative position of the body part to each of the MR images can be assessed. Thereby a motion of the body part can be detected.

Every time a new MR image may be acquired from which the perfusion is determined, the new MR image may be registered in view of a previous reference or to the initial MR image from which the baseline was acquired. This may serve to detect any large motion (beyond a user-defined threshold), which will likely lead to a large artefact in the perfusion and/or the change. It may be proposed to alert medical staff in this case to check the sedation depth manually or in any other way. Afterwards, a new baseline can be determined and the MR examination is continued.

According to an exemplary embodiment of the present invention, can further comprise the step updating a baseline of a time development of the perfusion based on the detected motion. Additionally, the method can comprise the step of updating at least one limitation of the change based on the updated baseline. Furthermore, the method can comprise the step of comparing the change to the at least one limitation of the change. The method can comprise the step of generating the signal indicative of the alteration of the sedation of the patient if the calculated change exceeds the at least one further limitation of the change.

In the case a motion is detected, a baseline can be updated and/or a further baseline can be defined to be able to determine the change of the perfusion. With the updated baseline, the perfusion can be determined taken into account the movement of the body part. Accordingly, a limitation needs to be updated. In the case the change of the perfusion exceeds the limitation the signal can be generated which is indicative for the alteration of the sedation of the patient. In other words, by detecting a movement of the body part the determination of the perfusion can be adapted.

According to an exemplary embodiment of the present invention, the method comprises the step of defining a region of interest in the first MR image based on the determined position of the body part within the first MR image. Additionally, the method can comprise the step of segmenting of a shell within the region of interest. The method can comprise the step of averaging perfusion values of vessels in the shell thereby determining the perfusion in the body part.

After the position of the body part is acquired, in which the perfusion should be determined, the shell in which for example the thermoregulatory vessels are located in needs to be located. Therefore, a region of interest is defined within the first MR image. Preferably, the body part is located in the region of interest such that parts of the MR image, which do not contain a part of the body part, can be removed, in order to save processing power. Furthermore, a shell can be segmented in the region of interest. To be able to detect a more reliable information of the perfusion within the shell, perfusion values of vessels in the shell thereby determining the perfusion in the body part.

According to an exemplary embodiment of the present invention, the method comprises the step of identifying the defined region of interest in the second MR image based on the determined motion of the body part. Additionally, the method can comprise the step of segmenting of a shell within the region of interest in the second MR image. Furthermore, the method can comprise the step of averaging perfusion values of vessels in the shell thereby determining the perfusion in the body part.

In other words, in case a movement of the body part is detected, the region of interest has to be moved accordingly. Additionally, it can be detected whether the orientation of the body part to the slice has changed, for example in the case a finger was bent. This can be detected for example, because in the first MR image the cross-section of the finger is round. In the case a motion has been detected because the finger was bent the cross-section of the finger, displayed in the second MR image, can be elliptical. Accordingly, the determining of the perfusion needs to be altered, for example by defining a new baseline.

According to an exemplary embodiment of the present invention, the body part is a peripheral body part of a human body, in particular a part of the human body selected from the group consisting of an arm, a leg, a nose, a hand, a finger, a toe and/or a foot.

In other words, all peripheral body parts of a human body can be used to perform this suggested method for sedation monitoring. This embodiment and the group of body parts have indeed the advantage that all body parts react very quickly to a change in sedation.

Sedation-induced skin perfusion changes can be most pronounced and at the same time well-accessible by small field-of-view and thus fast MR imaging in fingers, toes, and tip of nose. It maybe proposed to image perfusion in one of those regions, whichever is nearest to the region that is subjected to prescribed diagnostic MR imaging. The nose can be chosen for head and thoracic scans. The hands can be chosen for abdominal scans, and the feet may be chosen for scans of the lower periphery. Modern MR systems usually apply surface coil arrays that may cover large areas of the body in one go, so that signal reception in one of these options will typically be available. The image for determining the perfusion and its field-of-view can be chosen completely independent from that of the diagnostic scans. Two different embodiments for perfusion imaging in 2D slices and in 3D volumes may be proposed to address the trade-off between signal-to-noise-ratio (SNR), imaging time and complexity of image processing: 2D slices may have a shorter imaging time but provide lower signal. On the other hand they can be processed with relatively simple image processing. 3D volumes may take longer, cover more tissue and can be also intrinsically advantageous in terms of SNR, but they may require more elaborate image processing.

A second aspect of the invention is a computer program element, when executed on a processing unit, is capable of instructing the processing unit to perform the steps of:
- receiving an MR image of a body part,
- determining a perfusion of at least one part of the body part in the MR image,
- calculating a change of the perfusion of the at least one part of the body part,
- generating a signal indicative of an alteration of a sedation of the patient based on the calculated change of the perfusion of the at least one part of the body part.

In other words, the computer program element receives an MR image, which can be delivered by a storage device, e.g. a RAM, a hard disk, a SSD, a virtual RAM, a virtual hard disk, or any other storage device capable of storing MR images, a database, a network storage, a cloud provider, a PACS system or any other data storing system, which is capable of storing MR images. Alternatively, the computer program element can also receive MR images from a live MR image acquisition of the body part.

A third aspect of the invention is a computer readable medium capable of storing a computer program element as described previously and hereinafter.

The fourth aspect of the invention is an MR imaging device for sedation monitoring of a patient comprising:
- an MR image acquisition unit configured for acquiring MR images,
- a calculation unit,
- wherein the calculation unit is configured for determining a perfusion of at least one part of the body part in an acquired MR image,
- wherein the calculation unit is configured for calculating a change of the perfusion of the at least one part of the body part, and
- wherein the calculation unit is configured for generating a signal indicative of an alteration of a sedation of the patient based on the calculated change of the perfusion of the at least one part of the body part.

Exemplary, the MR imaging device comprises an MR acquisition unit able to acquire an MR image from a body part. Furthermore, the MR imaging device can comprise a calculation unit configured to be able to perform at least one step of the method as described previously and hereinafter.

These and other features of the invention will become apparent from and elucidated with reference to the embodiments described hereinafter.

### SHORT DESCRIPITION OF THE FIGURES

Fig. 1 shows a flowchart for illustrating steps of the method of evaluating MR images for sedation monitoring by MR imaging according to an embodiment of the invention.
Fig. 2 shows an MR imaging device according to an embodiment of the invention.

The reference symbols used in the drawings, and their meanings, are listed in summary form in the list of reference symbols. In principle, identical parts are provided with the same reference symbols in the figures.

### DETAILED DESCRIPTION OF EMBODIMENTS

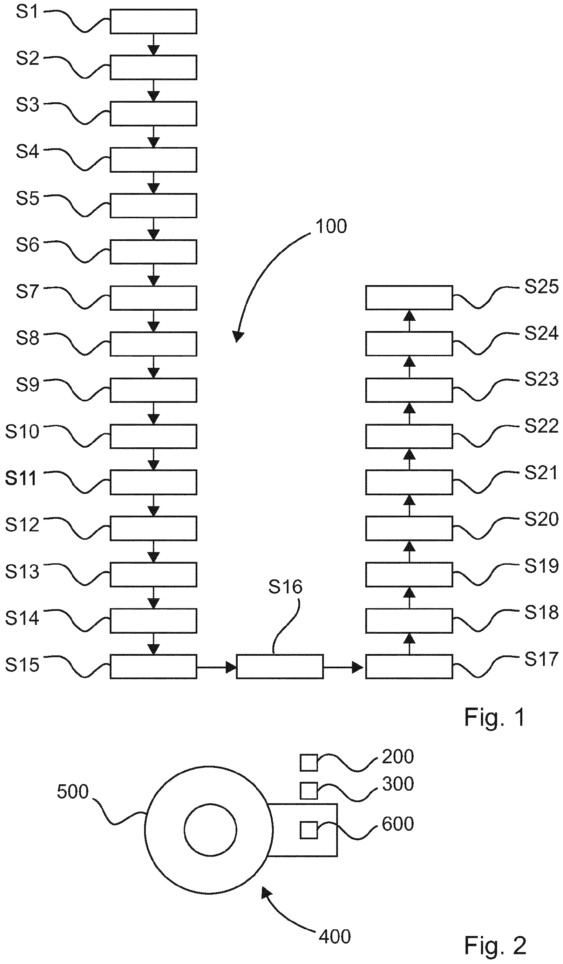

Fig. 1 shows a flowchart for illustrating the steps of the method 100 of evaluating MR images for sedation monitoring by MR imaging. The method 100 comprises the step S1 providing an MR image of a body part. The method 100 further comprises the step of determining S2 a perfusion of at least one part of the body part in the MR image. Additionally, the method 100 comprises the step of calculating S3 a change of the perfusion of the at least one part of the body part. Furthermore, the method 100 comprises the step of generating S4 a signal indicative of an alteration of a sedation of the patient based on the calculated change of the perfusion of the at least one part of the body part.

With the help of this embodiment, the perfusion can be determined by MR imaging and therefore tactile and verbal response, which has to be interpreted by medical staff during MR, imaging can be avoided and therefore cost can be saved. Additionally, no medically trained staff needs to be present in order to monitor the sedation of the patient since the method provides the teaching which is capable of indicating a signal if the sedation of the patient alters.

Additionally, the method 100 can comprise the steps S5-S24. All steps of the method 100 can be performed in sequence as presented or in any other sequence. Also, only single steps, bulks of steps and/or all steps of the steps S5-24 can be added to the steps of S1-S4.

Furthermore, the method 100 can comprise the step of generating S5 a baseline, which is a reference for determining the perfusion. Additionally, the method 100 can comprise the step defining S6 at least one limitation of the change based on the baseline. Furthermore, the method 100 can comprise the step of comparing S7 the change to the at least one limitation of the change. Furthermore, the method 100 can comprise the step of generating S8 the signal indicative of the alteration of the sedation of the patient if the calculated change exceeds at the at least one limitation of the change.

Furthermore, the method 100 can comprise the step of performing S9 blood oxygen level dependent imaging. Additionally, the method 100 can comprise the step of performing S10 arterial spin labelling. The method can further the step of segmenting the body part S11.The method can comprise the step of automatically detecting a motion of the body part by analyzing the sequence of the MR images S12. In particular, the method comprises a segmenting of the body part in the first MR image and in the second MR image thereby determining a position of the body part within the first MR image and the second MR image S13. Furthermore, the method can comprise a comparing of the position of the body part in first MR image to the position of body part in the second MR image S14. Additionally the method can comprise a generating of a signal indicative for a motion of the body part S15. In particular, the method comprises a updating of a baseline of a time development of the perfusion based on the detected motion updating S16. Furthermore, the method can comprise a updating of at least one limitation of the change based on the updated baseline S17. Additionally, the method can compare the change to the at least one further limitation of the change S18. The method can generate the signal indicative of the alteration of the sedation of the patient if the calculated change exceeds the at least one further limitation of the change S19. Additionally, the method can comprise a defining of a region of interest in the first MR image based on the determined position of the body part within the first MR image S20. Furthermore, the method can comprise a segmenting of a shell within the region of interest S21. The method can comprise an averaging of perfusion values of vessels in the shell thereby determining the perfusion in the body part S22. Additionally, the method can comprise the step of identifying the defined region of interest in the second MR image based on the determined motion of the body part S23. A segmenting of a shell within the region of interest in the second MR image S24 can be comprised by the method. Additionally, the method can comprise the step of averaging perfusion values of vessels in the shell thereby determining the perfusion in the body part S25.

Fig. 2 shows an MR imaging device 400, which comprises an MR acquisition unit 500 configured to acquire an MR image and a calculation unit 600 configured for determining a perfusion of at least one part of the body part in the MR image. Furthermore, the calculation unit 600 can be configured for calculating a change of the perfusion of the at least one part of the body part. Furthermore, the calculation unit 600 can be configured to generate a signal indicative of an alteration of the sedation of the patient based on the calculated change of the perfusion of the at least one part of the body part. Furthermore, a computer readable medium 300 can be comprised by the MR imaging device or can be held separately. Furthermore, a processing unit 200 can be comprised to the MR imaging device 400 or can be held separately.

Where an indefinite or definite article is used when referring to a singular noun, e.g. "a", "an" or "the", this includes a plurality of that noun unless something else is specifically stated. The terms "about" or "approximately" in the context of the present invention denote an interval of accuracy that the person skilled in the art will understand to still ensure the technical effect of the feature in question. The term typically indicates deviation from the indicated numerical value of ±20 %, preferably ±15 %, more preferably ±10 %, and even more preferably ±5 %.

Furthermore, the terms "first", "second", "third" or "(a)", "(b)", "(c)", "(d)" or "(i)", "(ii)", "(iii)", "(iv)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

### LIST OF REFERENCE SIGNS:

100 - Method of evaluating MR images for sedation monitoring by MR imaging
200 - processing unit
300 - computer readable medium
400 - MR imaging device
500 - MR acquisition unit
600 - calculation unit
S1 - providing an MR image
S2 - determining a perfusion
S3 - calculating a change
S4 - generating a signal
S5 - generating a baseline
S6 - defining at least one limitation
S7 - comparing the change
S8 - generating the signal
S9 - performing a blood oxygen level-dependent imaging
S10 - performing arterial spin labeling
S11 - segmenting the body part
S12 - automatically detecting a motion
S13 - segmenting the body part in the first MR image and in the second MR image
S14 - comparing the position of the body part
S15 - generating a signal indicative
S16 - updating a baseline
S17 - updating at least one limitation
S18 - comparing the change
S19 - generating the signal indicative
S20 - defining a region of interest
S21 - segmenting of a shell
S22 - averaging perfusion values of vessels
S23 - identifying the defined region of interest
S24 - segmenting of a shell
S25 - averaging perfusion values of vessels

## Claims

**1.** Method (100) of evaluating MR images for sedation monitoring of a patient by MR imaging, comprising the steps of:
- providing an MR image of a body part (S1),
- determining a perfusion of at least one part of the body part in the MR image (S2),
- calculating a change of the perfusion of the at least one part of the body part (S3),
- generating a signal indicative of the sedation of the patient based on the calculated change of the perfusion of the at least one part of the body part (S4).

**2.** Method according to claim 1, wherein the perfusion is determined in a thermoregulatory vessel, in particular an arterio-venos shunt, of the body part.

**3.** Method according to any of the preceding claims, wherein the perfusion is determined in a peripheral area of the body part, through which blood flows, in particular a tissue of the body part.

**4.** Method according to any of the preceding claims, wherein the determination of the perfusion in the body part is recorded over time, and wherein the step of determining the perfusion (S2) further comprises:
- determining a baseline of a time development of the perfusion, which is a reference for the determination of the perfusion (S5),
- defining at least one limitation of the change based on the baseline (S6),
- comparing the change to the at least one limitation of the change (S7),
- generating the signal indicative of the alteration of the sedation of the patient if the calculated change exceeds the at least one limitation of the change (S8).

**4.** Method according to any of the preceding claims, further comprising the step:
- performing blood oxygen level dependent imaging (BOLD) (S9), wherein a result of BOLD is interpreted in order to determine the perfusion.

**5.** Method according to any of the preceding claims, further comprising the step:
- performing arterial spin labeling (ASL) (S10) for the step of determining the perfusion (S2).

**6.** Method according to any of the preceding claims, wherein the provided MR image is a two dimensional MR image of the body part, and
wherein the two dimensional MR image is orientated perpendicular to a main orientation of the body part.

**7.** Method according to any of the preceding claims, wherein the provided MR image is a three dimensional MR image of the body part,
wherein the three dimensional MR image is orientated perpendicular to a main orientation, in particular in feet-head direction of a hand and/or a foot or in anterior-posterior direction of a nose.

**9.** Method according to any of the preceding claims, further comprising the step of:
- segmenting the body part (S11).

**10.** Method according to any of the preceding claims, wherein a sequence of MR images is provided, further comprising the steps of:
- automatically detecting a motion of the body part by analyzing the sequence of the MR images (S12).

**11.** Method according to claim 10,
wherein the sequence of MR images comprises at least a first MR image and a second MR image, further comprising the step of:
- segmenting the body part in the first MR image and in the second MR image thereby determining a position of the body part within the first MR image and the second MR image (S13),
- comparing the position of the body part in first MR image to the position of body part in the second MR image (S14), and
- generating a signal indicative for a motion of the body part (S15).

**12.** Method according to any of claims 10-11, further comprising the steps of:
- updating a baseline of a time development of the perfusion based on the detected motion (S16),
- updating at least one limitation of the change based on the updated baseline (S17),
- comparing the change to the at least one further limitation of the change (S18),
- generating the signal indicative of the alteration of the sedation of the patient if the calculated change exceeds the at least one further limitation of the change (S19).

**13.** Method according to any of claims 10-12, further comprising the steps of:
- defining a region of interest in the first MR image based on the determined position of the body part within the first MR image (S20),
- segmenting of a shell within the region of interest (S21),
- averaging perfusion values of vessels in the shell thereby determining the perfusion in the body part (S22).

**14.** Method according to any of claims 10-13, further comprising the steps of:
- identifying the defined region of interest in the second MR image based on the determined motion of the body part (S23),
- segmenting of a shell within the region of interest in the second MR image (S24),
- averaging perfusion values of vessels in the shell thereby determining the perfusion in the body part (S25).

**15.** Computer program element, which when executed on a processing unit, is configured to instruct the processing unit to perform the steps of:
- receiving an MR image of a body part,
- determining a perfusion of at least one part of the body part in the MR image,
- calculating a change of the perfusion of the at least one part of the body part,
- generating a signal indicative of an alteration of a sedation of a patient based on the calculated change of the perfusion of the at least one part of the body part.

**16.** A computer readable medium, on which a computer program element according to claim 15 is stored.

**17.** MR imaging device (400) for sedation monitoring of a patient comprising:
- an MR image acquisition unit (500) configured for acquiring MR images,
- a calculation unit (600),
- wherein the calculation unit (600) is configured for determining a perfusion of at least one part of the body part in an acquired MR image,
- wherein the calculation unit (600) is configured for calculating a change of the perfusion of the at least one part of the body part, and
- wherein the calculation unit (600) is configured for generating a signal indicative of an alteration of a sedation of the patient based on the calculated change of the perfusion of the at least one part of the body part.
